# EUROPEAN PATENT SPECIFICATION

(11) **EP 2 844 160 B1**
(45) Date of publication and mention of the grant of the patent: **21.11.2018**
(21) Application number: 13719146.6
(22) Date of filing: 24.04.2013
(51) Int. Cl.: A61B 17/15, A61B 17/17

(54) **SURGICAL TEMPLATES**
CHIRURGISCHE VORLAGEN
MATRICES CHIRURGICALES

(30) Priority: 24.04.2012 GB 201207180
(43) Date of publication of application: 11.03.2015
(73) Proprietor: Xiros Limited, Leeds LS19 7UE (GB)
(72) Inventor: SEEDHOM, Bahaa, Leeds Yorkshire LS19 7UE (GB); WHITE, Derrick, South Cave Yorkshire HU15 2AL (GB); TRESNAN, John, Pool-in-Wharfedale Yorkshire LS21 1TP (GB); HOGG, Alexander John, Leeds Yorkshire LS17 9BQ (GB)
(74) Representative: HGF Limited
(86) International application number: PCT/GB2013/051035
(87) International publication number: WO 2013/160674

(56) References cited:
- WO-A1-2009/001083
- US-A- 4 736 737
- US-A1- 2012 041 446

## Description

### Field of the Invention

The present invention relates to surgical template systems for use in working on bones, and in particular, although not exclusively, to surgical template systems for use in total knee replacement surgery.

### Background to the Invention

Surgical template systems for use in working on bones, and for preparing bones to receive prosthetics are known.

WO 2008/117028 discloses numerous surgical template systems providing advantages over other known surgical template systems. A number of the surgical template systems disclosed in that document comprise locating members having end surfaces adapted to conform to, and seat on, respective portions of a bone surface, those portions including portions of non-articular surfaces and portions of articular surfaces. The document discloses how these locating members, and their conformal locating member end surfaces may be patient specific, i.e. the surface of the bone to be worked on may be determined by suitable means, such as CT, CAT, or MRI scanning or imaging, and the locating means of the system may then be manufactured accordingly.

Although the template systems disclosed in WO 2008/117028 provide a number of advantages, certain problems remain to be addressed, as follows. The articular surface of a bone to be worked on may be ascertained (i.e. determined by a number of techniques, including CT, CAT, and MRI scanning), and patient specific locating means may be manufactured according to the ascertained articular surface, such that in the defined seated position for the template system, the relevant locating member surfaces conform to and seat on their respective portions of the bone articular surface. However, during surgery, it may become apparent that the articular surface is covered by cartilage, the condition of which may be difficult to determine pre-operatively. Thus, if the cartilage is left in place, a locating member manufactured to conform and sit on a respective portion of the bone articular surface may not sit in its planned position. Instead of sitting on the bone articular surface, it will sit on the cartilage surface, and hence the template system as a whole will not sit in the position required to bring its tool guiding aperture or apertures into the correct position with respect to the bone. An option in such circumstances is for the surgeon to remove the cartilage material from the relevant area such that a locating member adapted to sit on bone articular surface can indeed locate and sit on that surface. However, the need to remove cartilage complicates the surgical procedure, represents an additional task to be performed, and hence adds to the overall time required for the surgical procedure. Another disadvantage with having to remove cartilage to seat a locating member is that the cartilage may be in good condition, and depending on the particular surgical procedure being performed, it may be preferred, for other reasons, to leave that cartilage in place. A further problem is that removal of cartilage can itself be difficult.

In order to try to avoid the problem of having to remove cartilage to seat a locating member, one could consider ascertaining the cartilage surface itself before surgery, and then manufacturing the locating means to include a locating member having a seating surface adapted to conform with and seat on a respective portion of that cartilage surface, rather than an underlying articular surface of the bone. A problem with such an approach is, however, that certain scanning techniques, such as CT or CAT scanning, do not provide a suitable indication of cartilage surface, instead they produce an indication of just the bone surface. A further problem is that alternative techniques, such as MRI scanning, which can produce an indication of cartilage surface, may not provide sufficient information on the quality of that cartilage layer. Thus, whilst a template system with patient specific location means may be manufactured, with locating member surfaces adapted to seat on a predetermined cartilage surface, during surgery it may become apparent that the condition of the cartilage is unsuitable for seating the locating means. The cartilage may be degraded, damaged, diseased, or unsuitable in a variety of ways. For example, a diseased cartilage may not be sufficiently rigid to seat a patient specific template system in the desired, unique position.

Additional prior art surgical template systems, and their associated disadvantages, are described below.

WO 2004/017842 A2 discloses template systems for use in total knee replacement surgery. The template systems comprise an adjustable positioning block and a surgical tool guide. The systems are designed to allow further adjustment after placement on the bone and have mechanisms which allow this to occur. Disclosed positioning blocks sit on articular surfaces of bones, and this may lead to inaccuracies in placement. A disclosed adjustable positioning block comprises a tracker member which, in use, is tracked by a camera-based optical computer assisted surgery (CAS) system to assist the surgeon in correctly positioning the template system. Thus, use of the template systems entails adjustments of at least the positioning block in the operating theatre. This is disadvantageous because it is likely to increase both the complexity and time of the implantation procedure and adds to the number of intra-operative decisions to be made by the surgeon. Furthermore, the apparatus of the template system itself and the associated tracking equipment is complex and costly. Adjustable mechanisms are more expensive to manufacture than fixed, non-adjustable systems, are more prone to failure, and are harder to clean after use.

WO 2006/060795 A1 discloses a surgical template comprising an alignment guide, in the form of a mold having a surface for engaging a joint surface, and an instrument guide, comprising one or more tool-guiding apertures, that communicates with the mold. The mold (alignment guide) may be designed specifically for a given patient and is used to help orientate an instrument guide relative to the patient's anatomy. For knee surgery, each mold is adapted to conform to an articular surface of the femur or tibia. The instrument guide may be manufactured from a hard material and may be re-usable, whereas the alignment guide may be formed from a relatively soft material. Optional adjustment between the alignment device and the instrument guide during the surgical procedure is disclosed. Optional use of a metal insert in an opening in a plastic mold to accept a reamer or saw is disclosed. Where the instrument guide is positioned over the mold, such that a tool guided by the instrument guide needs to pass through the mold to reach the bone beneath, the document discloses the option of arranging for openings in the plastic mold (alignment guide), corresponding to the instrument guide opening positions, to be oversized to avoid introducing plastic debris into the joint being worked on. Disadvantages of the disclosed templates include the following. The custom (patient-specific) mold parts of both the femoral and tibial devices are adapted to conform with and sit on their articular surfaces. This can lead to inaccuracies in placement. During knee surgery the oscillating saw blade causes any device in contact with the joint to vibrate. In the disclosed systems in which the instrument guide blocks sit directly on top of the moulded parts, a problem is how to fasten the guide block to the mold so as not to come apart whilst the bone cuts are made. Positioning the instrument guide block on top of (over) a custom mold can move a guide aperture away from the bone surface, and can thus result in reduced accuracy when using that aperture to guide a cut. Furthermore, relatively thin (in terms of the depth of guide aperture provided) instrument guide blocks are disclosed, and by providing relatively shallow guide apertures, the accuracy of the bone cuts that can be made using those guide apertures if reduced. This is also exacerbated when the instrument guide block is located over the mold, such that a guided tool must also pass through the mold to reach the bone as the saw blade passes through the slits in them. The tibial and femoral devices sitting over the articular surfaces restrict the visibility of the surgeon whilst performing the bone cuts. A disclosed femoral mold, adapted to conform to the femoral articular surface, would appear to have to be made from flexible material to fit onto the end of the femur (which is, as a rough approximation, bell shaped). A flexible mold cannot be used to provide rigid location for an instrument guide block, and hence further inaccuracies in cutting the bone are introduced.

The paper "Computer-assisted Total Knee Arthroplasty Using Patient-specific Templating", M. A. Hafez et al, Clinical Orthopaedics and Related Research, No. 444, pp. 184-192 discloses femoral and tibial templates for total knee replacement surgery. This paper discloses one femoral and one tibial template, each customized to an individual bone by a process comprising scanning, and each then manufactured by a rapid prototyping technique. Each template is a one-piece block, comprising locators having surfaces adapted to seat the template in a unique position on the respective bone, and also comprising guide slots and holes to guide saw blades and drill bits to work on the bone. Each template is designed for single use (i.e. after that use it is disposed of). The paper discloses use of the templates to perform total knee arthroplasties on cadaveric and plastic knees only. The templates are produced from a Polyamide (nylon) composite material (DuraForm™ 3D Systems), which is a durable material for creating functional (tooling) prototypes. This material is licensed for in vivo exposure, i.e. coming into contact with tissue when used as an instrument, but not as an implant. Once manufactured, the templates are sterilized and ready for use. The paper demonstrates the usefulness and some of the advantages of patient specific templates. However, disadvantages of the disclosed templates include the following. The relatively soft material used to form each of the single unit templates, and which therefore forms the walls of the guide apertures, readily sheds particulate material when in contact with moving tool bits (e.g. saw blades, drill bits), which is unacceptable as it might in the long term have an undesirable toxic effect on the tissues of a patient. Further, the particulate matter might cause damage to the plastic prosthetic component if trapped between the two prosthetic components while they are in use. Friction between the moving surgical tools and the device can cause further shedding of particulate material from the device and would generate sufficient heat that can melt DuraForm™ under normal operating conditions. This melting of the device material can cause seizure of the cutting tool. DuraForm™ is porous. The inclusions in the material may be 'opened up' during surgery by the movement of powered surgical tools over its surface resulting in the release of more particulate material with the consequences stated above. It is now possible to rapid prototype customized devices using stainless steel resulting in nonporous devices. However, constructing each device as a single unit, for the purpose of single use is extremely expensive and this option is therefore not likely to be cost effective. Lastly, the templates disclosed in the paper are relatively bulky, and substantially reduce visibility of the femur and tibia being worked on.

Document WO2009/001083A1 discloses a patient specific surgical template system.

Embodiments of the present invention aim to obviate or mitigate at least one of the problems associated with the prior art.

### Summary of the Invention

According to a first aspect of the invention there is provided a surgical template system for use in working on a bone, comprising:
a tool guiding portion (which may also be described as a tool guide) comprising a guide aperture for receiving and guiding a tool to work on the bone; and
locating means (e.g. attached to the tool guiding portion) for locating the tool guiding portion at a predetermined (pre-ascertained, defined) position with respect to the bone such that the guide aperture has a fixed orientation (fixed position) with respect to the bone, the locating means comprising:
   a locating member having a first locating surface, the first locating surface being adapted to conform to either a predetermined (i.e. previously determined, ascertained, measured, or known) surface of cartilage covering a predetermined (i.e. previously determined, ascertained, measured, or known) articular surface of the bone or a predicted surface of cartilage covering, or assumed to be covering, the predetermined articular surface of the bone; and
   an adapter member (or adapter, or adapter assembly) having a second locating surface and being adapted to attach to the locating member in an operative position in which the second locating surface is adapted to engage the predetermined articular surface of the bone if the cartilage is removed or not present.

This first aspect provides the advantage that it is both patient specific, in that the locating surfaces are adapted to conform to and sit on surfaces associated with the particular patient bone to be worked on ascertained before surgery (so as to seat the locating means and tool guiding portion in the correct, unique position for the guide aperture to guide a tool), and adaptive to accommodate for cartilage condition discovered only during surgery. Thus, if the actual cartilage surface discovered during surgery corresponds to the predetermined or predicted cartilage surface and is in suitable condition, the adaptor member need not be used, and the locating member can be used to seat directly on the cartilage surface, the first locating surface conforming to and seating on a respective portion of the actual cartilage surface. However, if the anticipated cartilage is completely absent, the adaptor member can be attached to the locating member, so that the locating surface of the adaptor member can seat on the articular surface to seat the locating means and tool guiding portion in the correct, predetermined position with respect to the bone. Furthermore, if cartilage is discovered to be present during surgery, but its surface does not correspond to the predetermined or predicted surface, or if the cartilage is in a condition unsuitable for seating the first locating surface, then the surgeon can remove cartilage material to expose the underlying bone articular surface, and the adaptor member can be fitted to the locating member to seat the locating means and tool guiding portion on the bone by means of the adaptor member locating surface engaging the exposed articular surface.

In certain embodiments the locating means comprises a body portion attached to the tool guiding portion, and the locating member is arranged to extend from the body portion.

In certain embodiments the tool guiding portion comprises a tool guide block attached to the body portion. Advantageously, the tool guide block can be made from a relatively hard material, such as a metal, to avoid shedding problems. The locating means can be manufactured from a different material, such as a softer material.

In certain embodiments the tool guide block is attached to the body portion by non-releasable attachment means, for example a snap-fit mechanism.

In certain embodiments the locating member comprises an arm arranged to extend from the body portion.

In certain embodiments the adapter member is adapted to fit into a corresponding slot, recess, or aperture provided in the locating member.

In certain embodiments the adapter member is adapted to attach to the locating member in a unique orientation.

In certain embodiments the adapter member is an insert to the locating member.

In certain embodiments the adapter member is a cap adapted to cover the first locating surface.

In certain embodiments the adapter member is attached to the locating member by a hinge, the hinge permitting movement of the adapter member from the operative position, in which the adapter member may cover the first locating surface, to an inoperative position, in which the first locating surface is exposed for seating on the cartilage surface.

In certain embodiments the system further comprises breakable (e.g. frangible) attachment means attaching the adapter member to the locating member in the operative position, the breakable attachment means being breakable to allow movement of the adapter member from the operative position to expose the first locating surface.

In certain embodiments the system further comprises a snap-fit attachment mechanism for attaching the adapter member to the locating member in the operative position.

In certain embodiments the locating member is a first locating member, the adapter member is a first adapter member, and the system further comprises a second locating member having a locating surface adapted to conform to either the predetermined or predicted surface of cartilage, and a second adapter member having a locating surface and being adapted to attach to the second locating member in an operative position in which the locating surface of the second adapter member is adapted to engage the predetermined articular surface of the bone if the cartilage is removed or not present, such that the locating means locates the tool guiding portion in the predetermined position by means of one of the first locating member and first adapter member and one of the second locating member and second adapter member seating on the cartilage or articular surface.

In certain embodiments the first and second locating means and adapter members are adapted to at least inhibit mechanical attachment of the first adapter member to the second locating member and of the second adapter member to the first locating member. Thus, the first adaptor member may be adapted so that it fits only on the first locating means, not the second locating means, and the second adaptor member may be adapted so that it fits only on the second locating means, not the first locating means. Thus, during surgery, there is no possibility of an adaptor member being fitted to the wrong locating member, which would result in the locating means and tool guiding portion being seated in an incorrect position with respect to the bone.

Another aspect of the present invention provides a surgical template system for use in working on a bone, comprising:
a tool guiding portion comprising a guide aperture for receiving and guiding a tool to work on the bone; and
locating means for locating the tool guiding portion at a predetermined position with respect to the bone such that the guide aperture has a fixed orientation with respect to the bone, the locating means comprising:
   first and second interchangeable locating members, the first locating member having a first locating surface, the first locating surface being adapted to conform to either a predetermined surface of cartilage covering a predetermined articular surface of the bone or a predicted surface of cartilage covering, or assumed to be covering, the predetermined articular surface of the bone, and the second locating member having a second locating surface adapted to engage the predetermined articular surface of the bone if the cartilage is removed or not present.

This second aspect of the invention provides the same advantages as the first aspect of the invention, in that it is both patient specific and adaptive during surgery to account for different cartilage conditions, or the presence or absence of cartilage altogether, discovered only during surgery. In this aspect, rather than an adaptor member being attachable to the locating member, the surgeon has the option to use either the first or second interchangeable locating member, depending on whether the anticipated cartilage surface is present and suitable for seating the locating means, or not. In other words, if the anticipated cartilage is present and suitable (i.e. in suitable condition) the first locating member is used, its locating surface being seated on the cartilage surface to seat the locating means and tool guiding portion in the predetermined, required position. Alternatively, if cartilage is not present, or has to be removed by the surgeon, then the second interchangeable locating member is used, rather than the first locating member, such that the locating surface of the second locating member seats on the articular surface of the bone, yet still seats the locating means and tool guiding portion in the same desired, predetermined position with respect to the bone.

Features of embodiments of the first aspect may be employed in embodiments of the second aspect, with corresponding advantage.

In certain embodiments the locating means comprises a body portion attached to the tool guiding portion, and attachment means for attaching, for example releasably, either the first locating member or the second locating member to the body portion.

In certain embodiments each of the first and second locating members comprises a respective arm.

In certain embodiments the locating means comprises a second pair of interchangeable locating members having respective locating surfaces including a locating surface adapted to conform to either the predetermined or predicted surface of cartilage, and a locating surface adapted to engage the predetermined articular surface of the bone if the cartilage is removed or not present, such that the locating means locates the tool guiding portion in the predetermined position by means of one of each pair of interchangeable locating members seating on the cartilage or articular surface.

In certain embodiments the first locating surface is adapted to conform to and engage (seat on) a respective portion of the predetermined or predicted cartilage surface when the locating means is in a seated position in which the tool guiding portion is in said predetermined position.

In certain embodiments the second locating surface may be the surface of a tip, point, or other contact feature of the adapter, adapted to make contact with a minimal portion of the articular bone surface. In such embodiments, the second locating surface may not be adapted to conform to an extended portion of the articular surface; the second locating surface may not, on its own, be patient specific, but its position, when the adapter is attached to the locating member, is. In alternative embodiments, however, the second locating surface is adapted to conform to the predetermined articular surface, for example to conform to an area or portion of that surface.

In certain embodiments the second locating surface is adapted to conform to and engage (seat on) a respective portion of the predetermined articular surface when the locating means is in a seated position in which the tool guiding portion is in said predetermined position.

In certain embodiments at least the locating member or members and the adapter member or members are patient-specific.

In certain embodiments the locating means comprises at least one further locating member having a further locating surface adapted to engage a predetermined non-articular surface of the bone, such that the PST is located on the bone to be worked partly by means of the further locating member. In certain embodiments the further locating surface may be the surface of a tip, point, or other contact feature of the further locating member. In alternative embodiments, the further locating surface may be adapted to conform to the non-articular surface (i.e. it may be shaped to correspond and seat on a particular portion of that bone surface).

In certain embodiments the tool guiding portion comprises a tool guide block comprising said guide aperture, the locating means comprises a plurality of locating members, each member having a respective engaging surface (e.g. end surface) for positioning against a surface of the bone or cartilage, the plurality of locating members including said locating member or members, and the system further comprises attachment means for non-adjustably attaching the tool guide block to the locating means such that, when attached, the member engaging surfaces are secured in fixed position with respect to each other, for engaging different respective portions of the surface of the bone or cartilage, and the guide aperture is secured in a fixed position with respect to the engaging surfaces.

Another aspect of the invention provides a method of manufacturing a surgical template system for use in working on a bone, the method comprising:
ascertaining (determining) an articular surface of the bone; and
making (manufacturing) a surgical template system in accordance with any one of the above-mentioned aspects, wherein said predetermined articular surface is the ascertained articular surface.

In certain embodiments, the method further comprises: ascertaining (determining) a surface of cartilage covering the ascertained articular surface, wherein said predetermined surface of cartilage is the ascertained surface of cartilage, and the first locating surface is adapted to conform to the predetermined cartilage surface. For example, non-invasive ultrasound may be used to map and register soft tissue structures onto 3D bone models. Also, although certain CT scanners are not able to detect cartilage, certain other CT scanners, e.g. dual energy CT scanners, may be able to, and may be used in methods embodying the invention.

In certain embodiments, the method further comprises: predicting a surface of cartilage covering or assumed to be covering the ascertained articular surface (e.g. predicting according to at least the ascertained articular surface), and wherein the first locating surface conforms to the predicted surface. For example, the cartilage surface may be predicted by assuming a uniform thickness of cartilage covering the articular surface, or by a more sophisticated technique taking into account typical variations in cartilage thickness over the articular surface. Certain cartilage estimation techniques used in embodiments of the invention take into account bone shape and disease state. Furthermore, the thickness of cartilage in a normal joint exhibits regional variations, which have been studied in numerous publications, and therefore can be estimated with a degree of accuracy for the purposes of PST design in embodiments of the invention. In other words, certain methods embodying the invention predict the cartilage surface using data determined from studies of a plurality of joints. In a diseased joint considered for replacement, cartilage is frequently of a reduced thickness (by a process of wear), and can be very much softer than the normal cartilage. Also, it can be frequently absent, leaving the underlying bone completely denuded.

In certain embodiments the ascertaining of the articular surface of the bone comprises use of x-rays and/or an MRI scanner. For example, CT or CAT scanning may be performed.

In certain embodiments the ascertaining of the surface of cartilage comprises use of an MRI scanner.

Another aspect of the invention provides a surgical template system for use in working on a bone, comprising:
a tool guiding portion comprising a guide aperture for receiving and guiding a tool to work on the bone; and
locating means for locating the tool guiding portion with respect to the bone such that the guide aperture has a fixed orientation with respect to the bone, the locating means comprising:
   a locating member having a first locating surface, the first locating surface being adapted to locate on a surface of cartilage covering an articular surface of the bone; and
   an adapter member having a second locating surface and being adapted to attach to the locating member in an operative position in which the second locating surface is adapted to locate on the articular surface of the bone if the cartilage is removed or not present.

Another aspect of the invention provides a surgical template system for use in working on a bone, comprising:
a tool guiding portion comprising a guide aperture for receiving and guiding a tool to work on the bone; and
locating means for locating the tool guiding portion with respect to the bone such that the guide aperture has a fixed orientation with respect to the bone, the locating means comprising:
   first and second interchangeable locating members, the first locating member having a first locating surface, the first locating surface being adapted to locate on a surface of cartilage covering an articular surface of the bone, and the second locating member having a second locating surface adapted to locate on the articular surface of the bone if the cartilage is removed or not present.

Embodiments of these aspects may incorporate features of the previously described aspects with corresponding advantage. Also described herein is a method of working on a bone, the method comprising:
ascertaining an articular surface of the bone;
ascertaining a surface of cartilage covering the ascertained articular surface, or predicting a surface of cartilage covering or assumed to be covering the ascertained articular surface;
manufacturing a surgical template system in accordance with any one of the above-mentioned aspects or embodiments, wherein the predetermined articular surface is the ascertained articular surface and the predetermined surface of cartilage is the ascertained surface of cartilage; and
during surgery:
if cartilage is covering the articular surface and is suitable for seating the locating member, locating the locating means on the bone to locate the tool guiding portion in the predetermined position, with the first locating surface seated on the cartilage surface;
if no cartilage is covering the articular surface, attaching the adapter member to the locating member in the operative position and locating the locating means on the bone to locate the tool guiding portion in the predetermined position, with the second locating surface seated on the articular surface; and
if cartilage is at least partly covering the articular surface but is unsuitable for seating the locating member, removing at least some of the cartilage to expose at least a portion of the underlying articular surface, attaching the adapter member to the locating member in the operative position, and locating the locating means on the bone to locate the tool guiding portion in the predetermined position, with the second locating surface seated on the exposed articular surface.

Also described herein is a method of working on a bone, the method comprising:
ascertaining an articular surface of the bone;
ascertaining a surface of cartilage covering the ascertained articular surface, or predicting a surface of cartilage covering or assumed to be covering the ascertained articular surface;
manufacturing a surgical template system in accordance with any one of the above-mentioned aspects or embodiments, wherein the predetermined articular surface is the ascertained articular surface and the predetermined surface of cartilage is the ascertained surface of cartilage; and
during surgery:
if cartilage is covering the articular surface and is suitable for seating the first locating member, utilising the first locating member and locating the locating means on the bone to locate the tool guiding portion in the predetermined position, with the first locating surface seated on the cartilage surface;
if no cartilage is covering the articular surface, utilising the second location member instead of the first locating member and locating the locating means on the bone to locate the tool guiding portion in the predetermined position, with the second locating surface seated on the articular surface; and
if cartilage is at least partly covering the articular surface but is unsuitable for seating the first locating member, removing at least some of the cartilage to expose at least a portion of the underlying articular surface, utilising the second location member instead of the first locating member, and locating the locating means on the bone to locate the tool guiding portion in the predetermined position, with the second locating surface seated on the exposed articular surface.

Also described herein is a method of fitting a prosthesis to a bone, the method comprising:
manufacturing a surgical template system using a method in accordance with any one of the above-mentioned aspects or embodiments;
using the locating means to locate the tool guiding portion in the predetermined position with respect to the bone;
using the guide aperture to guide a tool to work on the bone to prepare the bone for receiving the prosthesis; and
fitting the prosthesis to the prepared bone.

In certain variations the method further comprises scanning a patient to determine the surface shape of at least the articular surface of the bone, and selecting the prosthesis from a plurality of prostheses.

Also described herein is a surgical method comprising:
manufacturing a surgical template system using a method in accordance with any one of the above-mentioned aspects or embodiments;
using the locating means to locate the tool guiding portion in the predetermined position with respect to the bone;
using the guide aperture to guide a tool to work on the bone.

Another aspect provides surgical apparatus comprising:
locating means for a surgical template system in accordance with any one of the above-mentioned aspects or embodiments; and
patient identification means providing an indication of the patient to whom said bone to be worked on belongs.

In certain embodiments, the surgical template system is supplied with the, or each, adapter pre-assembled, i.e. attached to its respective locating member in the operative position. Each adapter could then be removed if, during surgery, it was discovered that cartilage was absent or very worn and its residue was appropriate to remove.

In certain embodiments, the tool guiding portion comprises a tool guide block comprising said guide aperture, the locating means comprises a plurality of locating members, each member having a respective engaging surface (e.g. end surface) for positioning against a surface of the bone or cartilage, the plurality of locating members including said locating member or members, and the system futher comprises attachment means for non-adjustably attaching the tool guide block to the locating means such that, when attached, the member engaging surfaces are secured in fixed position with respect to each other, for engaging different respective portions of the surface of the bone or cartilage, and the guide aperture is secured in a fixed position with respect to the engaging surfaces. In such embodiments, as the locating means and tool guide block are separate from one another (and must be assembled and attached together to use the template) they can be manufactured separately and this provides a number of advantages. Firstly the tool guide block may be manufactured as a hard-wearing, reusable component that is not patient-specific. It can, for example, be manufactured from a metal, such that the guide apertures have hard metal surfaces which can guide a moving tool (such as a vibrating or reciprocating saw blade or rotating drill bit) without shedding material. The locating means can be made separately, by different techniques and, for example, using different material. For example, the locating means may be manufactured using rapid prototyping techniques so as to be patient-specific, based on a predetermined bone shape, that is a bone shape, topography or geometry that has been determined by a suitable technique on the patient, such as by scanning (which may also be referred to as imaging). Thus, a patient-specific system can be produced by rapid prototyping of just the locating means, and not the tool guide block. This helps speed up the process and also reduces costs. In other words, embodiments of the invention offer the advantage that just some parts of the template system need be patient-specific, with other parts being "standard" i.e. predetermined and possibly reusable components. Advantageously, as the guide aperture or apertures are provided in the tool guide block and not in the patient-specific "custom" locating means, material can be used for the locating means that is suited to the patient-specific manufacturing technique (for example it may be a relatively soft plastic) whilst avoiding the problem of locating means material shedding during use, as the tools are guided by the guide block apertures.

It will be appreciated that in certain embodiments the locating members of the locating means are rigid such that the assembled guide block and locating means seats securely on a bone to be worked on in a predetermined, defined position.

Certain embodiments comprise at least one locating member arranged to seat against a non-articular surface of the bone. This is advantageous, because in general the non-articular surface can be more precisely determined from scanning, and thus enables the template to be manufactured such that it locates on the bone in a substantially unique position.

In certain embodiments the tool guide block is formed from a first material and the locating means is formed from a second, different material, and the first material may be harder than the second material. For example, the tool guide block may be formed from a metal, and the locating means may be formed from a non-metallic material, such as a plastic.

The locating means may have been formed by a rapid prototyping technique, e.g. from a non-metallic or a metallic material. Although rapid prototyping in metal is currently expensive, only the patient-specific locating means would need to be formed in this way, not the tool guide block as well. Thus, rapid prototyping of just the locating means in metal could be performed, at lower cost than if a unitary guide block and locating means were produced with such a technique. However, in many embodiments, the locating means is rapidly prototyped in non-metallic material.

In certain embodiments at least one of the locating members is generally cylindrical (with circular, elliptical or other cross sectional geometry) and/ generally elongate. At least one of the locating members may be a locating finger.

In certain embodiments the locating means and guide block are arranged such that, when attached together, the/or each guide aperture is arranged to guide a tool so as to avoid the locating means. This avoids the shedding problem, even if the locating means is formed from a plastic or other relatively soft material.

The system may further comprise securing means for securing the attached locating means and tool guide block to a bone to be worked on. For example, at least one said member may comprise a bore extending through the member to the member's end surface, and the securing means may comprise a pin adapted to extend through the bore so as to be drivable into a bone surface to pin the member to the bone. Certain embodiments further comprise a sleeve arranged to line said bore, wherein the pin is adapted to extend through the sleeve. A plurality of said members (some or all) may comprise bores, and the securing means then comprises a corresponding plurality of pins.

In certain embodiments, the securing means comprises a bore extending through the tool guide block, and the system further comprises a pin adapted to extend through the guide block bore so as to be insertable into a bone surface to pin the guide block to the bone. The pin may be adapted so that it can be driven into the bone (for example with blows from a hammer or mallet). However, in alternative embodiments, the pin may be adapted to screw into the bone. As the tool guide block can be manufactured from material which does not pose any shedding problems when in contact with a moving (e.g. rotating) pin such embodiments of the invention can provide clear advantages. The use of one or more securing bores and corresponding pins in the tool guide block itself can be in addition to bores and pins on the locating means, or an alternative. Thus, in certain embodiments the tool guide block may be secured to the bone independently of the locating means. In certain examples, having secured the tool guide block in this way at a position determined initially by the locating means, the locating means may be removed.

In certain embodiments, at least one guide aperture comprises at least one slot for guiding a saw blade. At least one guide aperture may be a hole for guiding a drill bit, and in certain embodiments the tool guide block comprises a plurality of guide apertures.

The locating members may be separate components, and the relative positions of their end surfaces may only be defined when they are attached to the tool guide block. However, in certain other embodiments the locating means comprises a body portion with a plurality of the locating members attached to that body portion and extending from it to their respective end surfaces. The attachment means may then be adapted to attach the body portion to the tool guide block. The body portion and the locating members extending from it may be integral. By manufacturing this body portion and plurality of locating members as a single unit, this provides the advantage that the relative positions of the end surfaces of those members extending from the body portion are completely fixed, even before the body portion is attached to the tool guide block. Thus, their relative positions cannot be affected by any tolerances involved in attachment of the body portion to the guide block, and hence the integral body portion and locating members help seat the assembled template system in the desired, substantially unique position on the bone.

In certain embodiments the attachment means comprises a snap-fit mechanism. This may be arranged to allow the custom and re-usable parts to be securely and quickly attached to one another without the use of bolts or screws (or alternatively could be used in addition to bolts or screws). In certain examples, the snap-fit mechanism may be such that additional devices may be required to separate the two components once attached (or to bring the components together). In certain embodiments, in order to separate the custom part (locating means) from the re-useable part (tool guide block) it may be necessary to break the custom part (or at least some component, element or part of the snap-fit mechanism). It is conceivable that in further alternative embodiments, the locating means may be removable from the tool guide block in alternative ways, for example by dissolving the locating means material.

In certain embodiments the system is a femoral system (i.e. for working on a femur), in which the locating means is arranged such that, when attached to the tool guide block, the member end surfaces are positioned to seat the locating means and guide block in a predetermined position on a specific femur, by engaging at least one cartilage surface or articular surface of a portion of the femur. The tool guide block may then comprise a guide slot for guiding a saw blade to cut off an end portion of the femur to leave a sawn end surface when the block and locating means are seated in the predetermined position. The locating means may comprise an additional locating member having an end surface for positioning against a non-articular anterior surface (i.e. the cortical bone surface just above the trochlea) of the femur.

Other embodiments provide a tibial template system, in which the locating means is arranged such that, when attached to the tool guide block, a plurality of said member end surfaces are positioned to seat the locating means and guide block in a predetermined position on a specific tibia by engaging at least one cartilage surface or articular surface of the tibia. It may employ further locators to seat on non-articular surface portions as well.

In certain embodiments, the manufacturing method may comprise manufacturing the tool guide block from a first material (e.g. a metal) and manufacturing the locating means from a second, different material (e.g. using a rapid prototyping technique, such as a laser sintering process).

In certain embodiments the method further comprises manufacturing the locating means and guide block such that, when attached together, the/or each guide aperture is arranged to guide a tool so as to avoid the locating means. At least one member may be manufactured to comprise a bore, such that a pin can be driven through the bore into the bone surface to secure the locating means to the bone, and each guide aperture may then be arranged to guide a tool so as to avoid the or each pin driven into the bone through a respective bore. In certain embodiments each bore is arranged so as to be generally perpendicular to a respective portion of the surface of said bone against which the respective end surface is adapted to seat.

In certain embodiments, manufacturing the locating means comprises manufacturing a body portion and a plurality of said locating members attached to and extending from the body portion to their respective end surfaces, for example as an integral unit.

In certain embodiments the bone is a femur. In certain other embodiments the bone is a tibia.

The locating means may be manufactured such that, when attached to the tool guiding portion, a plurality of locating means surfaces are positioned to seat the locating means and tool guide in a predetermined position on the bone by engaging non-articular surface portions of the bone, and such that another plurality of locating member end surfaces are positioned to seat the locating means and guide block in a predetermined position by engaging articular surface portions of the bone.

In certain embodiments, the determining, or ascertaining, a surface shape of the bone or cartilage comprises non-invasive scanning of a patient.

In certain embodiments, the method may further comprise scanning a patient to determine the surface shape of the bone, and selecting the prosthesis from a plurality of prostheses. This plurality of prostheses may include prostheses having a variety of different sizes. The method may further comprise selecting a desired position for the prosthesis relative to the bone.

In certain embodiments the method further comprises forming a virtual model of the bone and manipulating a virtual model of the selected prosthesis relative to the bone virtual model to determine the desired position. The selected prosthesis in certain embodiments has a defined interior surface shape, and the method further comprises using the selected desired position and the defined interior surface shape to determine the position of each guide aperture when the assembled template is seated in the defined position on the bone.

The method may further comprise selecting a tool guide block from a plurality of tool guide blocks, the selected block comprising a plurality of guide apertures corresponding to said defined interior surface shape.

In certain embodiments, the patient identification means may be provided on the locating means, but in alternative embodiments the identification means may be linked to the locating means by linking means.

In certain embodiments the locating means comprises locating means for a template system for a femur and for a template system for a tibia for a specific knee joint of a particular patient.

In certain embodiments the locating means, patient identification means, and linking means have been manufactured together using a rapid prototyping technique.

In certain embodiments the surgical apparatus further comprises an indicator, manufactured together and integrally with the locating means, the indicator comprising at least one feature having a dimension measurable to check a manufacturing accuracy of the locating means. This indicator may, for example, be separable from the locating means before the locating means is used in surgery.

In certain embodiments, the apparatus further comprises a gauge adapted to engage the indicator and thus provide a quick check of the indicator dimensions and hence the accuracy of manufacture of the integrally formed locating means before use.

From the above summary, and the following description, it will be appreciated that embodiments of the invention provide a number of advantages, as follows. In certain embodiments, a template is essentially split into two components; a "standard" (i.e. non-patient specific) cutting block (guide block), which may be made of a biocompatible metal, is attached to a "customized" locating means (which may be a block), thus forming a unitary template. The customized component is designed so as to locate in the unique position for the bone cuts and holes to be made. By making the cutting (guide) block from a biocompatible metal the problem of shedding of potentially harmful particulate matter that is associated with DuraForm does not arise. Stainless steel may be used to manufacture the cutting block and is also robust enough so as to allow the use of the metallic component repeatedly. The locating means (which in certain examples may be described as orientating blocks) can be manufactured with rapid prototyping technologies and are intended for single use. This approach not only avoids the problems associated with the porosity of structures manufactured using rapid prototyping but is more cost effective. The optimal position of the prosthesis is determined precisely during the preoperative planning procedure. The preoperative position of the prosthesis determines the positing of the device relative to the patient's anatomy. Thus the operative procedure is less complex and may be achieved in a shorter time. Embodiments of the invention do not need, and so do not include, adjustable mechanisms, and so are less complex to manufacture, are user friendly and more cost effective. Locators with a relatively small cross sectional area may be used, located upon the tibial plateau with certain tibial device. However, the small cross-sectional area of these cylinders reduces the errors. Certain minimally invasive tibial devices embodying the invention avoid the articular surface of the tibia altogether. The separate components of certain femoral and tibial devices embodying the invention are securely fastened by means of a screw so as to form a single unitary device which will not vibrate apart during use. Convergent pins securely fasten the relevant device to the appropriate bone. Care can be taken during the preoperative planning procedure to place the locators perpendicular to the bone surface. In doing so any possible collisions with the saw blade/drill/adjacent pins are identified and rectified when it is appropriate to do so. In embodiments of the invention, the custom locating means does not sit between the guide block apertures and the bone, so it is possible to design the cutting to be of the required depth to ensure accurate cutting of the bone whilst keeping the overall size of the cutting blocks small. Template systems embodying the invention are designed to be fitted with both ease and accuracy in a unique position on the appropriate bone. The positioning of each template system can be tested in the laboratory, before surgery, e.g. virtually tested during the pre-operative planning procedure. Certain embodiments are designed specifically to increase the visibility the surgeon has of the knee whilst performing the bone cuts. This will not only increase the appeal of each device to the surgeon but also the safety of its use.

### Brief Description of the Drawings

Embodiments of the invention will now be described, by way of example only, with reference to the accompanying figures, of which:
Figs. 1-5 are schematic representations of surgical template systems, or parts of such systems, embodying the invention and positioned on bones to be worked on;
Figs. 6-16 illustrate further surgical template systems, or components of such systems, embodying the invention;
Fig. 17 illustrates components of another surgical template system embodying the invention;
Fig. 18 illustrates another template system embodying the invention and located on a bone to be worked on;
Figs. 19 and 20 illustrate components of surgical template systems embodying the invention; and
Fig. 21 is a highly schematic representation of another surgical template system embodying the invention and positioned with respect to a bone to be worked on.

### Detailed description of the preferred embodiments

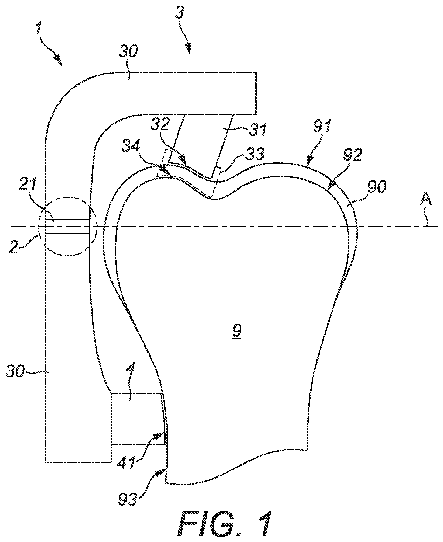

Referring now to Fig. 1, this shows a first patient specific, adaptive template system embodying the invention and positioned on a specific bone 9 to be worked on. The system comprises a tool guiding portion 2 comprising a guide aperture 21 which in this example is a slot for guiding a saw blade to perform a cut along the plane A indicated in the figure. In this example, the particular bone to be worked on 9 is a femur, although in other embodiments the bone may be a tibia, or any other bone having an articular surface normally covered by a layer of cartilage. The system also comprises locating means 3 attached to the tool guiding portion 2 (in this example the tool guiding portion and locating means are integral parts of a single body, although in alternative embodiments they may be separate components, attached together by suitable means). The locating means is patient specific, and adapted to seat on the bone 9 in a single, unique position in order to position the guide aperture 21 in a desired, predetermined position with respect to the bone so that the saw blade is guided to cut along the correct plane A. Although a single guide aperture 21 is shown in the figure, in alternative embodiments the tool guiding portion or portions may comprise a plurality of guide apertures. The locating means comprises a locating member 31 having a first locating surface 32 adapted to conform to a portion of the surface 91 of cartilage 90 covering the articular surface 92 of the bone 9. In this example, the cartilage surface 91 was ascertained before manufacturing the locating member 31 by suitable measurement techniques, for example by MRI scanning. In this example, the cartilage 90 is in a condition suitable for seating the locating member 31 and the components of the template system 1 are shown seated in the defined position on the bone, with the first locating surface 32 conforming to, engaging, and seated on a respective portion of the cartilage surface 91. However, it is possible that, during surgery, the cartilage 90 could have been found to be in a condition unsuitable for seating the locating member 31. Thus, the locating means also comprises an adaptor member 33 (which can also be described as an adaptor, or in alternative embodiments an adaptor assembly) having a second locating surface 34 and adapted to attach to the locating member in an operative position. Fig. 1 shows the adaptor 33 as a broken line, indicating the position of the adaptor 33 when attached to the locating member 31 in the operative position. In this attached position, the second locating surface 34 is adapted to engage the articular surface 92 of the bone. In this example, the second locating surface 34 is adapted to conform to a respective portion of the articular surface 92, that surface 92 having been ascertained by suitable means (e.g. CT or MRI scanning) before manufacture of the adaptor member 33. Thus, if the cartilage 90 were in poor condition, a surgeon could remove a portion of the cartilage to expose underlying articular surface 92, the adaptor 33 could be fitted on the locating member 31, and still locate the system in a correct position so that the cut plane A is at the correct position with respect to the bone 9. In this example, the locating means comprises a further locating member 4 having a locating surface 41 which is adapted to conform to, seat on, and engage a respective portion of a non-articular surface 93 of the bone when the template system is located in the desired position.

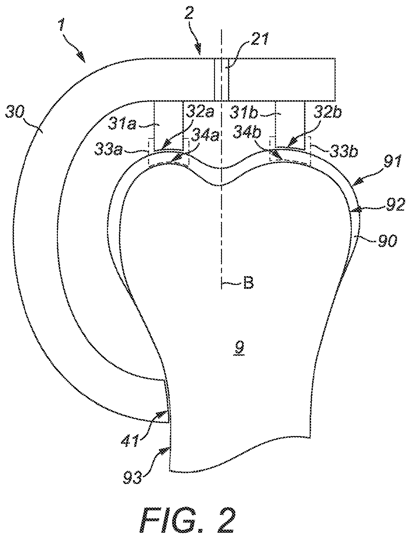

Referring now to Fig. 2, this shows an alternative template system where the guide aperture 21 is a bore to guide a drill bit to drill down into a bone 9 along axis B. The locating means comprises first and second locating members 31a, 31b, having respective locating end surfaces 32a, 32b, adapted to conform to, and locate on, respective portions of a predetermined cartilage surface 91. The locating means also comprises first and second adaptors 33a, 33b, having respective locating surfaces 34a, 34b for conforming to, engaging, and seating on respective portions of the bone articular surface 92. Thus, neither adaptor need be used if the cartilage surface corresponds to the predetermined or predicted surface 91. However, one or both of the adaptors 33a, 33b can be used if the cartilage 90 is found to be in unsuitable condition. The locating means comprises a body portion 30 which extends, in this example, around to a side surface of the bone, and has a further locating surface 41 adapted to conform and seat on a portion of a non-articular surface 93 of the bone 9.

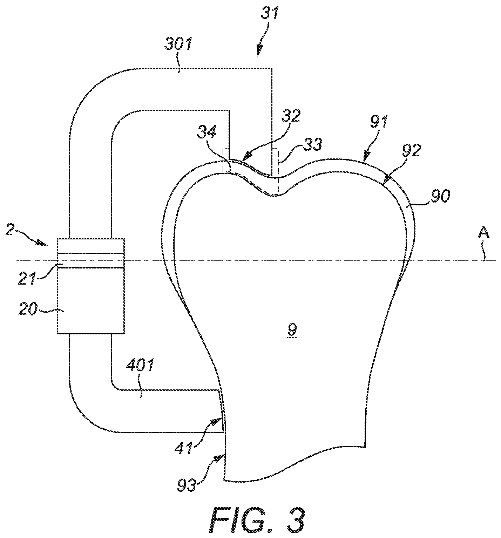

Moving on to Fig. 3, this shows an alternative embodiment, in which the tool guiding portion 2 comprises a tool guide block 20 having a guide aperture 21 in the form of a slot. The locating means comprises a locating member 31 in the form of a locating arm 301 having a locating end surface 32 adapted to conform to, engage, and seat on a respective portion of the cartilage surface 91. An adaptor member 33 is attachable to the end of the locating member 31 to provide a locating surface 34 for seating on the bone articular surface 92 if the cartilage 90 is in unsuitable condition. The locating arm 301 is attached to the tool guide block 20 by suitable attachment means. In certain embodiments, the attachment means may be releasable or non-releasable. However, the attachment means is arranged such that the attachment between the tool guide block 20 and locating arm 301 is not adjustable. In other words, the attachment arm 301 attaches to the block 20 in just a single, defined position and orientation such that, when attached, the locating surface 32 is completely fixed with respect to the cut axis A defined by the guide slot 21. The locating means comprises a further locating arm 401, attached to the guide block 20, and having a locating surface 41 adapted to conform to, seat on, and engage a non-articular surface 93 of the bone 9.

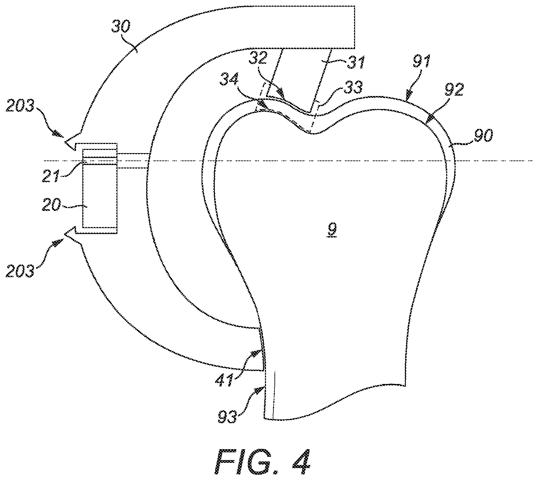

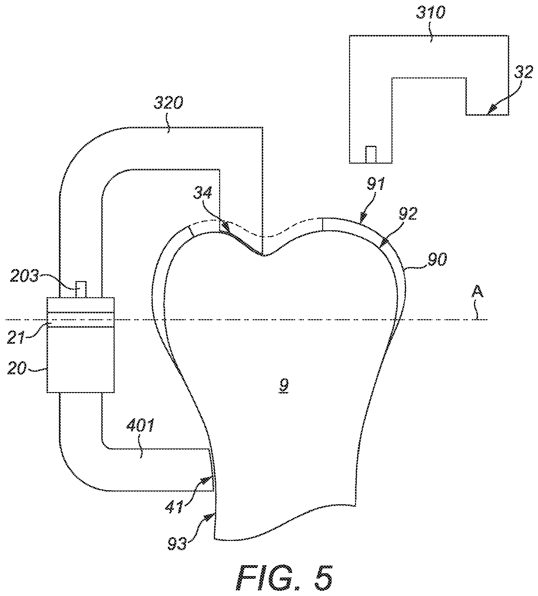

Referring now to Fig. 4, this shows an alternative adaptive patient specific template (PST) system embodying the invention. In this embodiment, the locating means comprises a locator body 30, and the tool guide portion comprises a guide block 20 formed from metal and comprising a guide slot 21. The guide block 20 is received in a recess in the body 30 and secured in place, in a non-adjustable, unique position, by means of a snap-fit mechanism 203. Clearances between the body 30 and block 20 are exaggerated in the figure for illustrative purposes only. The system comprises a locating member 31 and attachable adaptor 33 as described previously. Furthermore, the body 30 comprises another bone-engaging surface 41 as described above. Referring now to Fig. 5, this shows an alternative PST system embodying the invention. Here, rather than employing an adaptor 33, the system comprises a pair of interchangeable locating members 310, 320 in the form of interchangeable arms. Each of these arms 310, 320 is adapted to attach to a guide block 20 by means of non-adjustable attachment means 203. The first interchangeable arm 310 has a locating surface 32 adapted to conform to, seat on and engage a predetermined or predicted surface 91 of cartilage. The other arm 320 has a locating surface 34 adapted to conform to, seat on and engage the underlying bone articular surface 92. In Fig. 5, a portion of the cartilage 90 has been removed to expose underlying articular bone surface 92, and the second arm 320 has been attached to the block 20 to seat the assembly in the defined position, such that the cut plane A is precisely located with respect to the bone 9. The correct seating is achieved by means of the locating arm 320 and the further locating member 401 (in this example another locating arm) having conformal locating surface 41 sitting on bone non-articular surface 93.

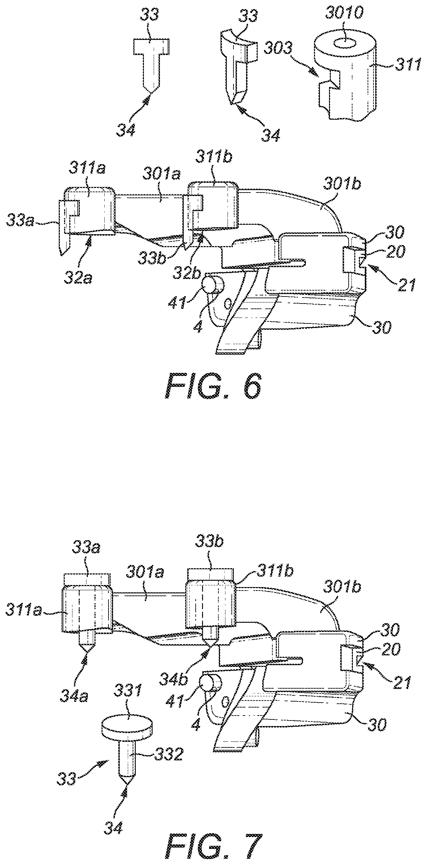

Referring now to Fig. 6, this shows another PST embodying the invention. In this example, the locating means comprises a body portion 30 attached to a guide block 20 having a guide slot 21. The locating means comprises first and second locating arms 301a and 301b, each extending from the body 30 to head portions 311a, 311b of these locating arms or members. Each head portion 311 has a locating surface 32 adapted to conform to a respective portion of a predetermined or predicted cartilage surface. First and second adaptors 33a, 33b are provided, each adapted to attach to a respective one of the heads 311. In this example, each adaptor 33 is generally T-shaped, having an end surface or locating tip 34 adapted to sit on the predetermined bone articular surface if the cartilage is removed or not present, when the adaptor 33 is inserted in a correspondingly shaped receiving slot 303 or recess provided in the locating member head portion 311. Each head portion 311 includes a bore 3010 through which an attachment pin can be driven to secure the PST in place on the bone when in the correct, seated position. Thus, in this example the locating surfaces 34 of the adaptors 33a, 33b are arranged, according to the pre-manufacture measurements, so as to correspond to and sit on the articular surface when the adaptors are attached to their respective locator member heads 311. These surfaces 34 of the adaptors in this example provide small contact areas, and need not necessarily be adapted to conform to the bone articular surface. In other words, they may essentially be points or tips. The template system of Fig. 6 includes at least one additional locating member 4 having a locating surface 41 adapted to engage a non-articular surface of the bone. It will be appreciated from Fig. 6 that each adaptor 33 is a press fit, single point adaptor. Each adaptor could be a reusable component, kept with a standard instrument kit. Alternatively, each adaptor could be a rapidly manufactured component, supplied with each PST. The recess 303 in each head portion 311 of the main PST body has a shoulder that sets the height of the respective adaptor to accurately locate on bone.

Referring to Fig. 7, this shows a system similar to that of Fig. 6. Here, each adaptor 33 is generally in the form of a pin, having a head portion 331 and a shaft portion 332 extending to a tip 34. Each adaptor 33 is a press fit, single point adaptor. Again, this could be a reusable component, kept with a standard instrument kit. Alternatively, the adaptor could be a rapid manufactured component, supplied with each PST. Each adaptor 33 is pressed or screwed in through a hole or bore 3010 in the PST up to the headed section 331, accurately setting the height of the tip 34 to locate on bone.

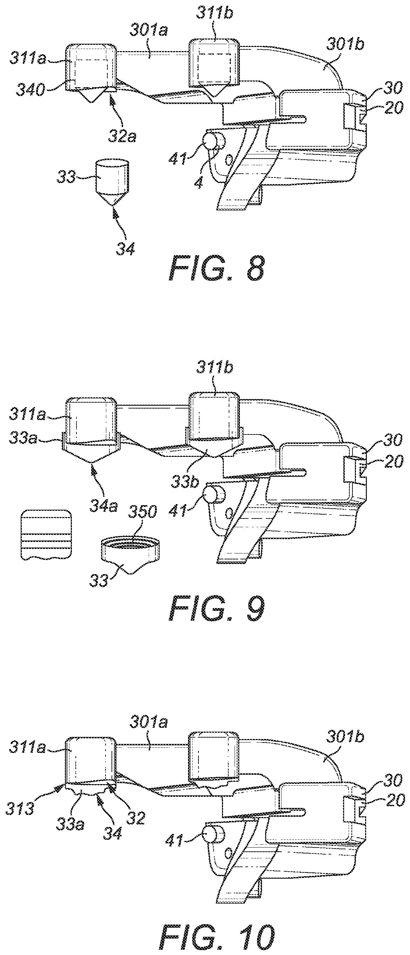

Referring now to Fig. 8, this shows another PST embodying the invention. Each adaptor 33 is pressed or screwed upwards into a recess 340 in the locating member head 311 up to a shoulder in that recess which accurately sets the height of the end or locating surface 34 of each adaptor to locate on bone.

Referring now to Fig. 9, this shows another PST embodying the invention. Here, each adaptor 33 is a threaded, single point adaptor, having an internal screw thread 350 for engaging a corresponding external screw thread provided on the locator member head. Each adaptor 33 is screwed or pushed onto a threaded portion of the PST to a stop that accurately sets the height of the bone-engaging tip 34a, 33b, to locate on bone.

Referring now to Fig. 10, this shows another PST system embodying the invention in which each adaptor 33a, 33b is attached to its respective locating member by means of a hinge 313. The adaptor 33a is attached by means of a hinge 313a to head portion 311a. The hinge 313 allows the adaptor 33a to be swung out of the way to expose the surface 32 to engage a cartilage surface. Alternatively, the adaptor can be left in, or moved to, the operative position shown in the figure for the surface 34 to engage the bone surface. The surface 34 could be a conforming surface to the bone, or in alternative embodiments may be a single point.

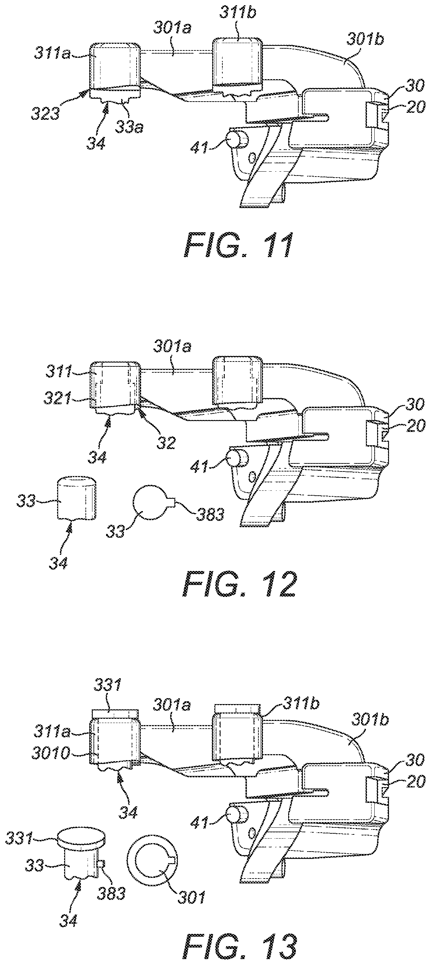

Referring now to Fig. 11, this shows another PST embodying the invention, in which each adaptor 33 is a snap-off adaptor. The adaptor 33a is supplied connected to the PST, and is formed onto the locating member head 311 at a distinct point or points. In other words, the adaptor 33 is attached to the head 311 by means of one or more breakable links. If the cartilage is found to be in suitable condition, and corresponds to the predicted or predetermined shape, then the surgeon does not require the bone locators 33, and these can be snapped off and discarded, exposing the conformal surfaces 32 underneath. If the cartilage is unsuitable and needs to be removed, or if the cartilage is not present, the bone adaptors 33 can be left in place, and then surfaces 34 may be conforming surfaces or single points for contacting the bone. In Fig. 11, just one breakable link is indicated 323, but in alternative embodiments more than one breakable link may be employed.

Referring now to Fig. 12, this shows another PST embodying the invention, in which each adaptor 33 is a press fit into the respective locating member, and has a conformal surface 34. Each adaptor 33 is pressed (or, in alternative embodiments, screwed, in which case the head 11 would need to be adapted to accommodate the protrusion as the adapter rotated) upwards into a recess in the respective locator head 311 up to a shoulder 321 that accurately sets the height to locate on bone. A protrusion 383 on the adaptor 33 is arranged to match with a corresponding cut out or slot in the recess in the head 311 to set the rotational position of the adaptor 33 relative to the head, at least when the adapter is in the operative position, or vice versa (in other words, a protrusion could be provided into the recess, to engage with a slot in the adaptor 33).

Referring to Fig. 13, this shows another PST embodying the invention employing press fit, conforming surface adaptors 33. Each adaptor 33 is pressed (or screwed, in alternative embodiments) in through a hole 3010 in each locating arm up to a head section 331, which accurately sets the height of the surfaces 34 to locate on bone. A protrusion 383 on each locator 33 is arranged to match with a cut out in the bore 3010 of each head 311 to set the rotational position (at least in the operative position), or vice versa in alternative embodiments.

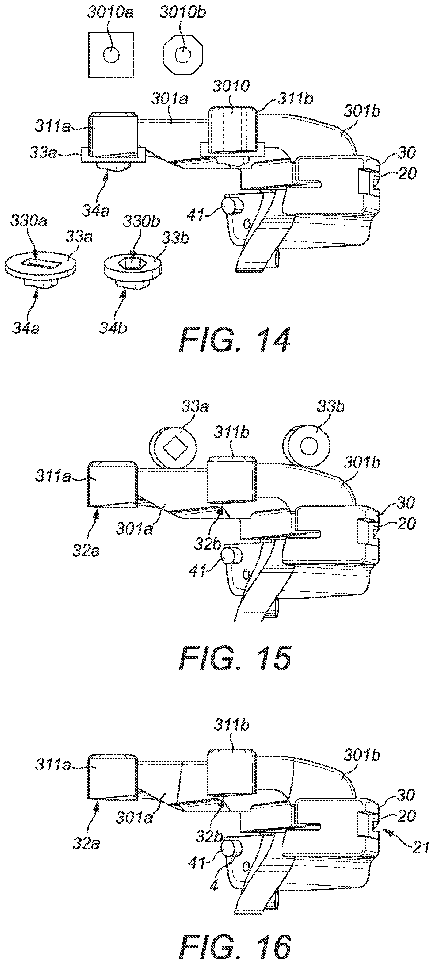

Referring now to Figure 14, this shows another PST embodying the invention employing press fit, conforming surface adaptors 33. These adaptors 33 are in the form of caps adapted to fit over and cover the end, locating surfaces of the locating arms. Each adaptor 33 is a pre-assembled end cap that is pressed onto the end surface of a respective one of the PST locating member heads. The adaptors and head portions utilise different shapes (such as a square or polygon as shown) to make a distinction between the medial and lateral locators so that they cannot be mixed up. In other words, the end of the locator head 311a is shaped to fit into a correspondingly shaped recess or socket 330a in the adapter 33a, but does not fit in the differently shaped recess 330b in the other adapter 33b. The locator head 311b is similarly shaped only to fit the adapter 33b. The locators can be removed, and also can be reattached, should the surgeon change his or her mind during the surgical process.

Referring now to Figure 15, this shows a similar PST to that shown in Figure 14. The adaptors 33 are press fit, conforming surface adaptors. The press fit adaptors 33 are initially moulded onto the arms 301 of the PST. These can be snapped off and press fitted to the locator heads 311 if required.

Referring now to Figure 16, this shows an alternative PST embodying the invention. This system comprises two pairs of interchangeable arms. One of each pair of interchangeable arms is shown attached to the locator body 30. The attached arms include a first arm 310a (of the first pair) having location surface 32a, and arm 310b (or the second pair) having location surface 32b. By changing part or all of the template arms it is possible to use a variety of locating parts. According to different embodiments, the arms or parts of the arms may be hinged, screwed, or pushed into place, or attached to the locator body by other means. They may have unique features to ensure that they are correctly seated on the correct template arm. They may be used in conjunction with other parts to ensure they are securely fastened.

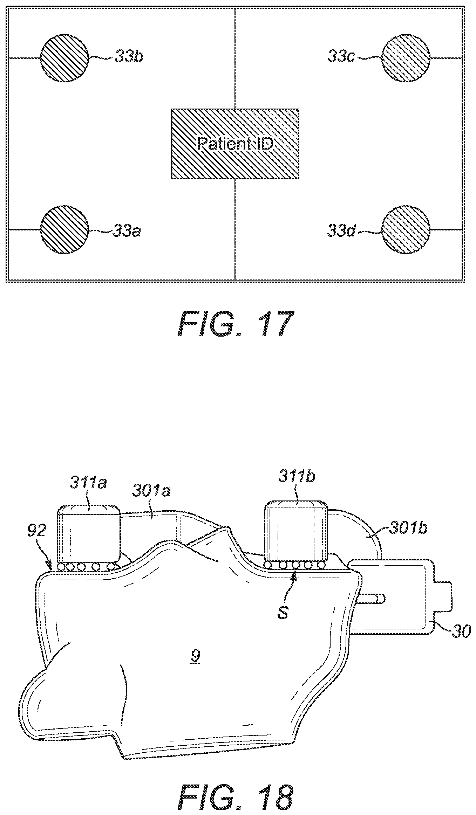

As will be appreciated, in certain embodiments the adaptor or adaptors are patient specific, and it may be desirable for each adaptor to carry an identification of the patient to whom it relates. Due to their size, however, it may not be possible to have the patient identification on every bone adapter. However it is still possible to manufacture all of the parts and the necessary patient identification as a single unit. In certain embodiments this single unit comprises the template body with the accessory bone adaptors attached to it. These could be snapped off, for example, as required. Alternatively, in other embodiments, the bone adapters (such as a plurality of bone adapters 33a-33d) may be manufactured as a separate single unit, as shown in fig. 17. This unit may, for example, be manufactured by rapid manufacturing. This single unit may also be fabric. In certain embodiments it is possible to manufacture this single unit using a variety of different manufacturing steps. These custom bone adapters may be patient specific or they may be generic parts that have been selected for a specific patient. Referring now to fig. 18, in some instances it may be desirable to have standard parts that can be used with the templates. These standard parts may or may not attach to the templates. These parts could be single use or they could be used many times. These could be metal, plastic, fabric, gel, putty or fluid filled for example. The parts may fully conform to the surface of the bone and act as a spacer of known thickness over an extended region of the bone or they may act as a spacer of known thickness in a small region of the template/bone. They may be formed from spheres S or have parts that have spherical features on them. The spacer or spacers could attach securely to either the template or bone, and may be bio absorbable.

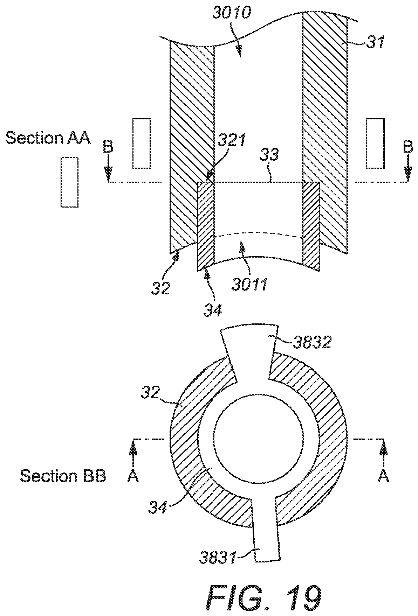

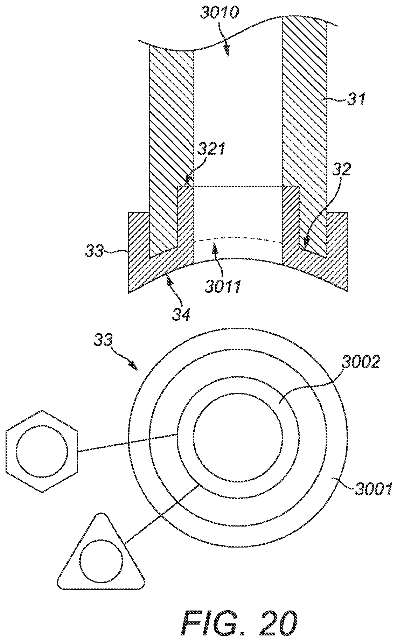

Referring now to Fig. 19, this shows detail of the attachment between an adaptor member, in the form of an insert 33, and a locating member 31 in certain embodiments of the invention. At least the end portion of the locator member 31 is generally cylindrical, and has a bore 3010. In certain embodiments the generally cylindrical portion has a circular outer surface in cross section, although in alternative embodiments the cylindrical portion may have a different outer shape (i.e. non-circular). The locator member 31 has an end locating surface 32 which is adapted to conform to a predetermined cartilage surface. The end of the bore 3010 adjacent this locating surface 32 is recessed to permit insertion of the adaptor insert 33. This recess includes a shoulder 321 which sets the position of the adaptor end surface 34 when the adaptor is fully inserted. The adaptor end surface 34 in this example is conformal, in that it is adapted to conform to a portion of the bone articular surface. The adaptor 33 also has an internal bore 3011, which has the same diameter as the bore 3010. Thus, when the adaptor 33 is inserted, a smooth bore of uniform diameter is provided, through which an attachment pin may be driven into the bone to secure the locator member to it. The adaptor or insert 33 in this example has a generally cylindrical wall, and the adaptor 33 also comprises a projection 3831 which projects from this outside wall of the insert and snugly fits into a correspondingly shaped and dimensioned groove in the wall of the locator. This projection 3831 defines the insert's rotational orientation with respect to the locator 31, and hence ensures that the conformal surface 34 is correctly orientated with respect to the bone to be worked on. In this particular embodiment, the adaptor 33 also includes a second projection 3832 of a different shape and size to the first projection 3831 to locate in a correspondingly shaped and sized further recess in the end of the locator 31. Thus, in this and other embodiments, two projections of different dimensions may be used to facilitate removal of the insert if, during surgery, it became apparent that sound (i.e. suitable) cartilage was present. Thus, the template system could be provided with the insert initially in place in the locator 31, and the insert could be removed if cartilage was present and suitable for use in locating the PST. Moving on to Fig. 20, this shows details of the attachment between another adaptor 33, in the form of a cap, and a locating member 31 in certain embodiments of the invention. As with the embodiments shown in Fig. 19, the locator 31 is generally cylindrical. The adaptor 33 is in the form of a cap having a conformal locating surface 34 for seating on bone. The cap 33 is adapted to fit over the end of the locator, engaging, seating on, and covering the locator end surface 32. The cap 33 comprises an inner annular portion 3002 which engages an inner surface of the bore 3010 of the locator and sits against a shoulder 321. This inner annular portion 3002 defines a bore 3011 which again is of the same diameter as the bore 3010. The cap 33 also comprises an outer annular portion 3001 which fits around and engages an outer annular surface of the generally cylindrical locator 31. The inner annular portion 3002 and the locator recess in which this portion is engaged are shaped so as to correspond to one another and permit attachment of the cap to the locator 31 in just a single, defined position. In embodiments where the PST comprises a further locator member and adaptor 33, the different adaptors 33 in the form of caps can have differently shaped inner annular portions 3002 so that each one can only be fitted onto its respective, correct locator. As will be appreciated, a wide variety of different shapes for the inner annular portion 3002 can be employed, i.e. shapes of different cross sections, such as triangular or polygonal. Furthermore, it will be appreciated that in alternative embodiments the inner annular portion and/or the outer annular portion 3001 may be shaped, together with the locator end surface, to ensure that the cap can only be fitted to the correct locator, and in the correct rotational orientation.

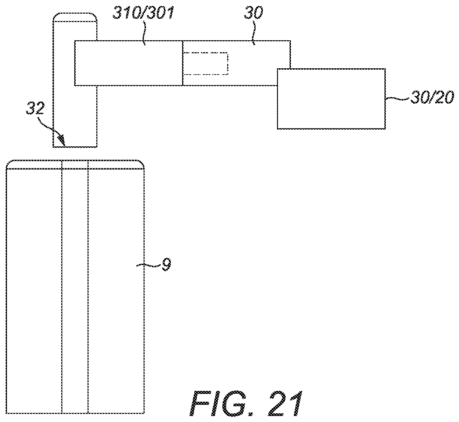

Referring now to Fig. 21, this is a highly schematic representation of part of another PST embodying the invention positioned close to a bone 9 to be worked on. A first interchangeable arm 310 is shown attached to the template body 30, but its locating surface 32 is not in contact with cartilage because the cartilage covering the articular surface has been eroded. The surgeon will therefore need to replace the template arm 310 with the other template arm of the pair, that other arm having an end surface 34 at a position which will engage the articular bone surface. Thus, due to cartilage wear, a locator designed to fit on cartilage surface may hover above the articular surface. Thus, in certain embodiments the cartilage locating member needs to be replaced by the bone locating member, or the cartilage locating member needs to be fitted with the bone adaptor to ensure that the PST is securely and uniquely located on the bone.

It will be appreciated that in certain template systems a plurality of locating member end surfaces may be adapted to be in contact with respective portions of a non-articular surface of the bone when the attached locating means and tool guide block are seated in the desired, defined position. In certain systems not embodying the invention, all of the locating members are arranged to seat against non-articular surfaces of the bone. In general, the non-articular surface can be more precisely determined from scanning. In embodiments of the invention, the system comprises at least one locating member having a locating (e.g. end) surface adapted to seat on cartilage covering an articular surface of the bone.

As will be appreciated, certain CAT scans do not show the topography of cartilage articular surfaces. Therefore, a CAT scan - based PST design with locators intended to rest on articular cartilage in accordance with certain embodiments is designed taking into account an estimated thickness of cartilage in the regions of contact between said locators and the articular cartilage. A difficulty arises when articular cartilage is worn out or totally absent in said regions. In such cases no contact is possible between the locator(s) and the articular surface; in the absence of adaption, the locators(s) will hover above the surface. It may not be cost effective to design two sets of PSTs, from which to select the set that suits the condition of the articular surfaces, which can only be, ascertained intra-operatively. Embodiments of the invention provide a solution that addresses this situation. Where it is not clear whether cartilage is present or absent in a regions of contact with a locator, in certain embodiments a locator is designed as if it is intended to rest on cartilage. In anticipation of the possibility of the cartilage being eroded thus leaving a gap between the locator and the bone surface, a separate component (an adapter member, for example in the form of a cap) is manufactured, to be mounted onto the end of that locator in certain embodiments. The thickness of that cap may be arranged so as to be equivalent to thickness of the missing cartilage. In certain embodiments the surface of the cap in contact with the locator conforms to the surface of the locator, and its surface in contact with the bone is designed to conform to that of the bone surface it comes into contact with. In the case where a residue of cartilage is present in such region it must be scraped with scalpel. The end of the cap is adapted to fit in or on the locator securely and in the correct orientation. In embodiments in which the PST has more than one locator that is intended to rest on cartilage, each of the adaptors (additional caps) preferably has unique mounting means onto its respective locator. Thus one locator end could be triangular fitting into a corresponding triangular cavity in the cap. Another locator's end might be polygonal, etc. The caps in certain embodiments are small components with insufficient surfaces to include the unique ID of the patient, and these various locators may be produced with the same rapid manufacturing process as the rest of the patient-specific components of the PSTs. The caps may be connected, with thin connecting arms, to a plate with the patients unique ID, and can be easily detached from the plate, if needed, during surgery. In alternative embodiments the caps may be supplied assembled onto their respective locators. During surgery, if cartilage was present in the regions of contact between locators and joint surface, the caps may be removed. Otherwise they are left in place, if the cartilage is absent in said regions. Certain alternative embodiments provide a cavity at the wall of a locator, to which a thin elongate component (adaptor) is clipped on, the component being adapted to locate on the bone in the region where cartilage is absent. The component may be made with a rapid manufacturing process, and may be reusable or a single use component.

It will be appreciated that joint articular surfaces, such as in the hip or knee, are those surfaces that come into contact with one another other during movement. In a healthy joint, these contacting surfaces will typically be surfaces of the layers of articular cartilage (layers of tough gristle, which are also referred to as cartilage in this document) covering the underlying bone material. Articular cartilage is distinct from the menisci in the knee joint, which do not adhere to bone, unlike articular cartilage. In this document the term "articular surface of the bone" refers to the surface of the bone material itself in the joint, i.e. a surface that would normally underlie (i.e. be covered with) articular cartilage.

Certain embodiments of the invention may be used in the field of total knee replacement (TKR) surgery. In one example of TKR surgery, two different assemblies each embodying the invention are used, one for use with the femur the other with the tibia, to aid the surgeon in cutting both of these bones to receive prosthetic knee components.

From the above description it will be appreciated that certain embodiments of the present invention relate to methods, systems and devices for facilitating total knee replacement surgery, particularly in respect of making the bone cuts in the knee bones so these are made to allow the prosthetic components to be implanted accurately and in the correct orientation within the joint of the recipient. Certain embodiments of the present invention comprise template systems that are customised for total knee replacement surgery via large incisions as well as those for minimally invasive surgery in which it is aimed to prepare the bone cuts and implant the prosthetic components through the smallest possible incisions in the joint. This reduces the trauma to the surrounding tissues, and speeds up the recovery of the patient.

## Claims

1. A patient specific surgical template system (1) for use in working on a specific bone of a specific patient, comprising:
a tool guiding portion comprising a guide aperture (21) for receiving and guiding a tool to work on the bone; and
patient specific locating means for locating the tool guiding portion at a predetermined position with respect to the bone such that the guide aperture has a fixed orientation with respect to the bone, the locating means comprising:
a patient specific locating member (31) having a first locating surface (32), the first locating surface being adapted to conform to either a predetermined surface of cartilage covering a predetermined articular surface of the bone or a predicted surface of cartilage covering, or assumed to be covering, the predetermined articular surface of the bone; and
a patient specific adapter member (33) having a second locating surface and being adapted to attach to the locating member in an operative position in which the second locating surface is adapted to engage the predetermined articular surface of the bone if the cartilage is removed or not present.

2. A system in accordance with claim 1, wherein the adapter member (33) is adapted to attach to the locating member in a unique orientation.

3. A system in accordance with any preceding claim, wherein the adapter member (33) is attached to the locating member by a hinge, the hinge permitting movement of the adapter member from the operative position, in which the adapter member may cover the first locating surface, to an inoperative position, in which the first locating surface is exposed for seating on the cartilage surface.

4. A system in accordance with any preceding claim, further comprising breakable attachment means attaching the adapter member to the locating member in the operative position, the breakable attachment means being breakable to allow movement of the adapter member from the operative position to expose the first locating surface.

5. A system in accordance with any preceding claim, further comprising a snap-fit attachment mechanism for attaching the adapter member to the locating member in the operative position.

6. A system in accordance with any preceding claim, wherein the locating member (31) is a first locating member, the adapter member is a first adapter member, and the system further comprises a second patient specific locating member having a locating surface adapted to conform to either the predetermined or predicted surface of cartilage, and a second patient specific adapter member having a locating surface and being adapted to attach to the second locating member in an operative position in which the locating surface of the second adapter member is adapted to engage the predetermined articular surface of the bone if the cartilage is removed or not present, such that the locating means locates the tool guiding portion in the predetermined position by means of one of the first locating member and first adapter member and one of the second locating member and second adapter member seating on the cartilage or articular surface.

7. A system in accordance with any preceding claim, wherein the first locating surface (32) is adapted to conform to and engage a respective portion of the predetermined or predicted cartilage surface when the locating means is in a seated position in which the tool guiding portion is in said predetermined position.

8. A system in accordance with any preceding claim, wherein the second locating surface is adapted to conform to the predetermined articular surface.

9. A system in accordance with claim 8, wherein the second locating surface is adapted to conform to and engage a respective portion of the predetermined articular surface when the locating means is in a seated position in which the tool guiding portion is in said predetermined position.

10. A system in accordance with any preceding claim, wherein the locating means comprises at least one further locating member having a further locating surface adapted to conform to a predetermined non-articular surface of the bone.

11. A method of manufacturing a patient specific surgical template system for use in working on a specific bone of a specific patient, the method comprising:
ascertaining an articular surface of the bone; and
making a patient specific surgical template system (1) in accordance with any one of claims 1 to 10, wherein said predetermined articular surface is the ascertained articular surface.

12. A method in accordance with claim 11, further comprising:
ascertaining a surface of cartilage covering the ascertained articular surface, wherein said predetermined surface of cartilage is the ascertained surface of cartilage, and the first locating surface is adapted to conform to the predetermined cartilage surface.

13. A method in accordance with claim 11, further comprising:
predicting a surface of cartilage covering or assumed to be covering the ascertained articular surface, and wherein the first locating surface conforms to the predicted surface.

14. A method in accordance with any one of claims 11 to13, wherein said ascertaining of the articular surface of the bone comprises use of x-rays and/or an MRI scanner.

15. A method in accordance with any one of claims 11 to14, wherein said ascertaining of the surface of cartilage comprises use of an MRI scanner.

## Patentansprüche

1. Patientenspezifisches chirurgisches Vorlagesystem (1) zur Verwendung beim Arbeiten an einem spezifischen Knochen eines spezifischen Patienten, das Folgendes umfasst:
einen Werkzeugführungsabschnitt, der eine Führungsöffnung (21) zum Aufnehmen und Führen eines Werkzeugs zum Arbeiten an dem Knochen umfasst; und
patientenspezifische Positionierungsmittel zum Positionieren des Werkzeugführungsabschnitts an einer vorbestimmten Position hinsichtlich des Knochens, sodass die Führungsöffnung eine feste Ausrichtung hinsichtlich des Knochens aufweist, wobei die Positionierungsmittel Folgendes umfassen:
ein patientenspezifisches Positionierungselement (31), das eine erste Positionierungsfläche (32) aufweist, wobei die erste Positionierungsfläche dazu angepasst ist, entweder einer vorbestimmen Knorpelfläche, die eine vorbestimmte Gelenkfläche des Knochens bedeckt, oder einer vorhergesagten Knorpelfläche, die die vorbestimmte Gelenkfläche des Knochens bedeckt, oder vermutlich bedeckt, zu entsprechen; und
ein patientenspezifisches Adapterelement (33), das eine zweite Positionierungsfläche aufweist und dazu angepasst ist, sich in einer Arbeitsposition, in der die zweite Positionierungsfläche angepasst ist, um die vorbestimmte Gelenkfläche des Knochens in Eingriff zu nehmen, wenn der Knorpel entfernt oder nicht vorhanden ist, an dem Positionierungselement zu befestigen.

2. System nach Anspruch 1, wobei das Adapterelement (33) dazu angepasst ist, sich in einer einzigartigen Ausrichtung an dem Positionierungselement zu befestigen.

3. System nach einem der vorhergehenden Ansprüche, wobei das Adapterelement (33) durch ein Scharnier an dem Positionierungselement befestigt ist, wobei das Scharnier eine Bewegung des Adapterelements von der Arbeitsposition, in der das Adapterelement die erste Positionierungsfläche bedecken kann, in eine Nichtarbeitsposition, in der die erste Positionierungsfläche freiliegt, um auf der Knorpelfläche aufzuliegen, gestattet.

4. System nach einem der vorhergehenden Ansprüche, ferner umfassend zerbrechliche Befestigungsmittel, die das Adapterelement an dem Positionierungselement in der Arbeitsposition befestigen, wobei die zerbrechlichen Befestigungsmittel zerbrechlich sind, um eine Bewegung des Adapterelements aus der Arbeitsposition zu ermöglichen, um die erste Positionierungsfläche freizulegen.

5. System nach einem der vorhergehenden Ansprüche, ferner umfassend einen Schnappbefestigungsmechanismus zum Befestigen des Adapterelements an dem Positionierungselement in der Arbeitsposition.

6. System nach einem der vorhergehenden Ansprüche, wobei das Positionierungselement (31) ein erstes Positionierungselement ist, wobei das Adapterelement ein erstes Adapterelement ist, und wobei das System ferner ein zweites patientenspezifisches Positionierungselement umfasst, das eine zweite Positionierungsfläche aufweist, die dazu angepasst ist, entweder der vorbestimmten oder vorhergesagten Knorpelfläche zu entsprechen, und wobei ein zweites patientenspezifisches Adapterelement eine Positionierungsfläche aufweist und dazu angepasst ist, sich in einer Arbeitsposition an dem zweiten Positionierungselement zu befestigen, in der die Positionierungsfläche des zweiten Adapterelements dazu angepasst ist, die vorbestimmte Gelenkfläche des Knochens in Eingriff zu nehmen, wenn der Knorpel entfernt oder nicht vorhanden ist, sodass die Positionierungsmittel den Werkzeugführungsabschnitt durch eines von dem ersten Positionierungselement und dem ersten Adapterelement und eines von dem zweiten Positionierungselement und dem zweiten Adapterelement, das auf der Knorpel- oder Gelenkfläche aufliegt, in der vorbestimmten Position positionieren.

7. System nach einem der vorhergehenden Ansprüche, wobei die erste Positionierungsfläche (32) dazu angepasst ist, einem jeweiligen Abschnitt der vorbestimmten oder vorhergesagten Knorpelfläche zu entsprechen und diesen in Eingriff zu nehmen, wenn das Positionierungsmittel sich in einer aufliegenden Position befindet, in der der Werkzeugführungsabschnitt sich in der vorbestimmten Position befindet.

8. System nach einem der vorhergehenden Ansprüche, wobei die zweite Positionierungsfläche dazu angepasst ist, der vorbestimmten Gelenkfläche zu entsprechen.

9. System nach Anspruch 8, wobei die zweite Positionierungsfläche dazu angepasst ist, einem jeweiligen Abschnitt der vorbestimmten Gelenkfläche zu entsprechen und diesen in Eingriff zu nehmen, wenn das Positionierungsmittel sich in einer aufliegenden Position befindet, in der der Werkzeugführungsabschnitt sich in der vorbestimmten Position befindet.

10. System nach einem der vorhergehenden Ansprüche, wobei das Positionierungsmittel wenigstens ein weiteres Positionierungselement umfasst, das eine weitere Positionierungsfläche aufweist, die dazu angepasst ist, einer vorbestimmten Nichtgelenkfläche des Knochens zu entsprechen.

11. Verfahren zur Herstellung eines patientenspezifischen chirurgischen Vorlagesystems zur Verwendung beim Arbeiten an einem spezifischen Knochen eines spezifischen Patienten, wobei das Verfahren Folgendes umfasst:
Ermitteln einer Gelenkfläche des Knochens und Anfertigen eines patientenspezifischen chirurgischen Vorlagesystems (1) nach einem der Ansprüche 1 bis 10, wobei die vorbestimmte Gelenkfläche die ermittelte Gelenkfläche ist.

12. Verfahren nach Anspruch 11, ferner umfassend:
Ermitteln einer Knorpelfläche, die die ermittelte Gelenkfläche bedeckt, wobei die vorbestimmte Knorpelfläche die ermittelte Knorpelfläche ist, und wobei die erste Positionierungsfläche dazu angepasst ist, der vorbestimmten Knorpelfläche zu entsprechen.

13. Verfahren nach Anspruch 11, ferner umfassend:
Vorhersagen einer Knorpelfläche, die die ermittelte Gelenkfläche bedeckt oder vermutlich bedeckt, und wobei die erste Positionierungsfläche der vorhergesagten Fläche entspricht.

14. Verfahren nach einem der Ansprüche 11 bis 13, wobei das Ermitteln der Gelenkfläche des Knochens die Verwendung von Röntgenstrahlen und/oder einem MRT-Scanner umfasst.

15. Verfahren nach einem der Ansprüche 11 bis 14, wobei das Ermitteln der Knorpelfläche die Verwendung eines MRT-Scanners umfasst.

## Revendications

1. Système de matrice chirurgicale (1) spécifique à un patient pour utilisation dans une intervention sur un os spécifique d'un patient spécifique, comprenant :
une partie de guidage d'outil comprenant une ouverture de guide (21) pour recevoir et guider un outil pour intervenir sur l'os ; et
un moyen de positionnement spécifique à un patient pour positionner la partie de guidage d'outil au niveau d'une position prédéfinie par rapport à l'os de sorte que l'ouverture de guide présente une orientation fixe par rapport à l'os, le moyen de positionnement comprenant :
un élément de positionnement (31) spécifique à un patient présentant une première surface de positionnement (32), la première surface de positionnement étant conçue pour s'adapter soit à une surface prédéfinie de cartilage recouvrant une surface articulaire prédéfinie de l'os soit à une surface prévue de cartilage recouvrant, ou supposée recouvrir, la surface articulaire prédéfinie de l'os ; et
un élément adaptateur (33) spécifique à un patient comportant une seconde surface de positionnement et étant conçu pour se fixer à l'élément de positionnement dans une position opérationnelle dans laquelle la seconde surface de positionnement est conçue pour venir au contact de la surface articulaire prédéfinie de l'os si le cartilage est enlevé ou n'est pas présent.

2. Système selon la revendication 1, ledit élément adaptateur (33) étant conçu pour se fixer à l'élément de positionnement dans une orientation unique.

3. Système selon l'une quelconque des revendications précédentes, ledit élément adaptateur (33) étant fixé à l'élément de positionnement par une charnière, la charnière permettant le mouvement de l'élément adaptateur de la position opérationnelle, dans laquelle l'élément adaptateur peut recouvrir la première surface de positionnement, à la position non opérationnelle, dans laquelle la première surface de positionnement est exposée pour reposer sur la surface de cartilage.

4. Système selon l'une quelconque des revendications précédentes, comprenant en outre un moyen de fixation frangible fixant l'élément adaptateur à l'élément de positionnement dans la position opérationnelle, le moyen de fixation frangible étant frangible pour permettre le mouvement de de l'élément adaptateur à partie de la position opérationnelle pour exposer la première surface de positionnement.

5. Système selon l'une quelconque des revendications précédentes, comprenant en outre un mécanisme de fixation encliquetable pour fixer l'élément adaptateur à l'élément de positionnement dans la position opérationnelle.

6. Système selon l'une quelconque des revendications précédentes, ledit élément de positionnement (31) étant un premier élément de positionnement, ledit élément adaptateur étant un premier élément adaptateur, et ledit système comprenant en outre un second élément de positionnement spécifique à un patient comportant une surface de positionnement conçue pour s'adapter à la surface de cartilage soit prédéfinie soit prévue, et un second élément adaptateur spécifique à un patient comportant une surface de positionnement et étant conçu pour se fixer à au second élément de positionnement dans une position opérationnelle dans laquelle la surface de positionnement du second élément de positionnement est conçue pour venir au contact de la surface articulaire prédéfinie de l'os si le cartilage est enlevé ou n'est pas présent, de sorte que le moyen de positionnement positionne la partie de guidage d'outil dans la position prédéfinie au moyen de l'un du premier élément de positionnement et du premier élément adaptateur et de l'un du second élément de positionnement et du second élément adaptateur reposant sur la surface de cartilage ou articulaire.

7. Système selon l'une quelconque des revendications précédentes, ladite première surface de positionnement (32) étant conçue pour s'adapter à et venir au contact d'une partie respective de la surface de cartilage prédéfinie ou prévue lorsque le moyen de positionnement est dans une position de logement dans laquelle la partie de guidage d'outil se trouve dans ladite position prédéfinie.

8. Système selon l'une quelconque des revendications précédentes, ladite seconde surface de positionnement étant conçue pour s'adapter à la surface articulaire prédéfinie.

9. Système selon la revendication 8, ladite seconde surface de positionnement étant conçue pour s'adapter à et venir au contact d'une partie respective de la surface articulaire prédéfinie lorsque le moyen de positionnement se trouve dans une position de logement dans laquelle la partie de guidage d'outil se trouve dans ladite position prédéfinie.

10. Système selon l'une quelconque des revendications précédentes, ledit moyen de positionnement comprenant au moins un élément de positionnement supplémentaire comportant une surface de positionnement supplémentaire conçue pour s'adapter à une surface non articulaire prédéfinie de l'os.

11. Procédé de fabrication d'un système de matrice chirurgicale spécifique à un patient pour utilisation dans une intervention sur un os spécifique d'un patient spécifique, le procédé comprenant :
la vérification d'une surface articulaire de l'os ; et
la fabrication d'un système de matrice chirurgicale (1) spécifique à un patient selon l'une quelconque des revendications 1 à 10, ladite surface articulaire prédéfinie étant la surface articulaire vérifiée.

12. Procédé selon la revendication 11, comprenant en outre :
la vérification d'une surface de cartilage recouvrant la surface articulaire vérifiée, ladite surface prédéfinie de cartilage étant la surface vérifiée de cartilage, et la première surface de positionnement étant conçue pour s'adapter à la surface de cartilage prédéfinie.

13. Procédé selon la revendication 11, comprenant en outre :
la prévision d'une surface de cartilage recouvrant ou supposée recouvrir la surface articulaire vérifiée, et ladite première surface de positionnement s'adaptant à la surface prévue.

14. Procédé selon l'une quelconque des revendications 11 à 13, ladite vérification de la surface articulaire de l'os comprenant l'utilisation de rayons X et/ou d'un scanner IRM.

15. Procédé selon l'une quelconque des revendications 11 à 14, ladite vérification de la surface de cartilage comprenant l'utilisation d'un scanner IRM.
